# EUROPEAN PATENT APPLICATION

(11) **EP 3 476 940 A2**
(43) Date of publication of application: **01.05.2019**
(21) Application number: 18201973.7
(22) Date of filing: 23.10.2018
(51) Int. Cl.: C12N 15/113

(54) **RNA INTERFERENCE MEDIATED THERAPY FOR NEURODEGENERATIVE DISEASES**

(30) Priority: 25.10.2017 IT 201700121288
(71) Applicant: Università Degli Studi Di Torino, 10124 Torino (IT)
(72) Inventor: BRUSCO, Alfredo, 10137 Torino (IT); GIORGIO, Elisa, 10024 Moncalieri (TO) (IT)
(74) Representative: Freyria Fava, Cristina

(57) **Abstract**

Therapeutic agent for use in the treatment of neurodegenerative diseases associated with lamin B1 protein overexpression, wherein said therapeutic agent comprises one or more siRNAs targeting one lamin B1 allele.

SEQUENCE LISTING

<110>  Università degli Studi di Torino

<120>  RNA interference madiated therapy for neurodegenerative diseases

<130>  BIT20069-CF

<160>  86

<170>  PatentIn version 3.5

<210>  1
<211>  19
<212>  DNA
<213>  artificial

<220>
<223>  ASP-C1  s

<400>  1
uaauaacugu guacuguuc                                                    19


<210>  2
<211>  19
<212>  DNA
<213>  artificial

<220>
<223>  ASP-C1 AS

<400>  2
auuauugaca caugacaag                                                    19


<210>  3
<211>  19
<212>  DNA
<213>  ARTIFICIAL

<220>
<223>  ASP-C2 S

<400>  3
aauaacugug uacuguucg                                                    19


<210>  4
<211>  19
<212>  DNA
<213>  ARTIFICIAL

<220>
<223>  ASP-C2 AS

<400>  4
uuauugacac augacaagc                                                    19


<210>  5
<211>  19
<212>  DNA

<213>  ARTIFICIAL

<220>
<223>  ASP-C3 S

<400>  5
auaacugugu acuguucgg                                                    19


<210>  6
<211>  19
<212>  DNA
<213>  ARTIFICIAL

<220>
<223>  ASP-C3 AS

<400>  6
uauugacaca ugacaagcc                                                    19


<210>  7
<211>  19
<212>  DNA
<213>  ARTIFICIAL

<220>
<223>  ASP-C4 S

<400>  7
uaacugugua cuguucgga                                                    19


<210>  8
<211>  19
<212>  DNA
<213>  ARTIFICIAL

<220>
<223>  ASP-C4 AS

<400>  8
auugacacau gacaagccu                                                    19


<210>  9
<211>  19
<212>  DNA
<213>  ARTIFICIAL

<220>
<223>  ASP-C5 S

<400>  9
aacuguguac uguucggaa                                                    19


<210>  10
<211>  19
<212>  DNA
<213>  ARTIFICIAL

<220>

<223>  ASP-C5 AS

<400>  10
uugacacaug acaagccuu                                                    19


<210>  11
<211>  19
<212>  DNA
<213>  ARTIFICIAL

<220>
<223>  ASP-C6 S

<400>  11
acuguguacu guucggaag                                                    19


<210>  12
<211>  19
<212>  DNA
<213>  ARTIFICIAL

<220>
<223>  ASP-C6 AS

<400>  12
ugacacauga caagccuuc                                                    19


<210>  13
<211>  19
<212>  DNA
<213>  ARTIFICIAL

<220>
<223>  ASP-C7 S

<400>  13
cuguguacug uucggaagg                                                    19


<210>  14
<211>  19
<212>  DNA
<213>  ARTIFICIAL

<220>
<223>  ASP-C7 AS

<400>  14
gacacaugac aagccuucc                                                    19


<210>  15
<211>  19
<212>  DNA
<213>  ARTIFICIAL

<220>
<223>  ASP-C8 S

<400>  15

uguguacugu ucggaaggg                                                    19

<210>  16
<211>  19
<212>  DNA
<213>  ARTIFICIAL

<220>
<223>  ASP-C8 AS

<400>  16
acacaugaca agccuuccc                                                    19

<210>  17
<211>  19
<212>  DNA
<213>  ARTIFICIAL

<220>
<223>  ASP-C9 S

<400>  17
guguacuguu cggaagggg                                                    19

<210>  18
<211>  19
<212>  DNA
<213>  ARTIFICIAL

<220>
<223>  ASP-C9 AS

<400>  18
cacaugacaa gccuucccc                                                    19

<210>  19
<211>  19
<212>  DNA
<213>  ARTIFICIAL

<220>
<223>  ASP-C10 S

<400>  19
uguacuguuc ggaaggggu                                                    19

<210>  20
<211>  19
<212>  DNA
<213>  ARTIFICIAL

<220>
<223>  ASP-C10 AS

<400>  20
acaugacaag ccuucccca                                                    19

<210>  21
<211>  19
<212>  DNA
<213>  ARTIFICIAL

<220>
<223>  ASP-C11 S

<400>  21
guacuguucg gaagggguu                                                    19


<210>  22
<211>  19
<212>  DNA
<213>  ARTIFICIAL

<220>
<223>  ASP-C11 AS

<400>  22
caugacaagc cuuccccaa                                                    19


<210>  23
<211>  19
<212>  DNA
<213>  ARTIFICIAL

<220>
<223>  ASP-C12 S

<400>  23
uacuguucgg aagggguuc                                                    19


<210>  24
<211>  19
<212>  DNA
<213>  ARTIFICIAL

<220>
<223>  ASP-C12 AS

<400>  24
augacaagcc uuccccaag                                                    19


<210>  25
<211>  19
<212>  DNA
<213>  ARTIFICIAL

<220>
<223>  ASP-C13 S

<400>  25
acuguucgga agggguucc                                                    19


<210>  26
<211>  19
<212>  DNA

<213>  ARTIFICIAL

<220>
<223>  ASP-C13 AS

<400>  26
ugacaagccu uccccaagg                                                    19


<210>  27
<211>  19
<212>  DNA
<213>  ARTIFICIAL

<220>
<223>  ASP-C14 S

<400>  27
cuguucggaa gggguuccu                                                    19


<210>  28
<211>  19
<212>  DNA
<213>  ARTIFICIAL

<220>
<223>  ASP-C14 AS

<400>  28
gacaagccuu ccccaagga                                                    19


<210>  29
<211>  19
<212>  DNA
<213>  ARTIFICIAL

<220>
<223>  ASP-C15 S

<400>  29
uguucggaag ggguuccuc                                                    19


<210>  30
<211>  19
<212>  DNA
<213>  ARTIFICIAL

<220>
<223>  ASP-C15 AS

<400>  30
acaagccuuc cccaaggag                                                    19


<210>  31
<211>  19
<212>  DNA
<213>  ARTIFICIAL

<220>

<223>  ASP-C16 S

<400>  31
guucggaagg gguuccuca                                                    19


<210>  32
<211>  19
<212>  DNA
<213>  ARTIFICIAL

<220>
<223>  ASP-C16 AS

<400>  32
caagccuucc ccaaggagu                                                    19


<210>  33
<211>  19
<212>  DNA
<213>  ARTIFICIAL

<220>
<223>  ASP-C17 S

<400>  33
uucggaaggg guuccucaa                                                    19


<210>  34
<211>  19
<212>  DNA
<213>  ARTIFICIAL

<220>
<223>  ASP-C17 AS

<400>  34
aagccuuccc caaggaguu                                                    19


<210>  35
<211>  19
<212>  DNA
<213>  ARTIFICIAL

<220>
<223>  ASP-C18 S

<400>  35
ucggaagggg uuccucaaa                                                    19


<210>  36
<211>  19
<212>  DNA
<213>  ARTIFICIAL

<220>
<223>  ASP-C18 AS

<400>  36

agccuucccc aaggaguuu                                                    19

<210>  37
<211>  19
<212>  DNA
<213>  ARTIFICIAL

<220>
<223>  ASP-C19 S

<400>  37
cggaaggggu uccucaaau                                                    19

<210>  38
<211>  19
<212>  DNA
<213>  ARTIFICIAL

<220>
<223>  ASP-C19 AS

<400>  38
gccuucccca aggaguuua                                                    19

<210>  39
<211>  19
<212>  DNA
<213>  ARTIFICIAL

<220>
<223>  ASP-T1 S

<400>  39
uaauaacugu guacuguut                                                    19

<210>  40
<211>  19
<212>  DNA
<213>  ARTIFICIAL

<220>
<223>  ASP-T1 AS

<400>  40
auuauugaca caugacaaa                                                    19

<210>  41
<211>  19
<212>  DNA
<213>  ARTIFICIAL

<220>
<223>  ASP-T2 S

<400>  41
aauaacugug uacuguutg                                                    19

<210>  42
<211>  19
<212>  DNA
<213>  ARTIFICIAL

<220>
<223>  ASP-T2 AS

<400>  42
uuauugacac augacaaac                                                    19


<210>  43
<211>  19
<212>  DNA
<213>  ARTIFICIAL

<220>
<223>  ASP-T3 S

<400>  43
auaacugugu acuguutgg                                                    19


<210>  44
<211>  19
<212>  DNA
<213>  ARTIFICIAL

<220>
<223>  ASP-T3 AS

<400>  44
uauugacaca ugacaaacc                                                    19


<210>  45
<211>  19
<212>  DNA
<213>  ARTIFICIAL

<220>
<223>  ASP-T4 S

<400>  45
uaacugugua cuguutgga                                                    19


<210>  46
<211>  19
<212>  DNA
<213>  ARTIFICIAL

<220>
<223>  ASP-T4 AS

<400>  46
auugacacau gacaaaccu                                                    19


<210>  47
<211>  19
<212>  DNA

<213>  ARTIFICIAL

<220>
<223>  ASP-T5 S

<400>  47
aacuguguac uguutggaa                                                    19


<210>  48
<211>  19
<212>  DNA
<213>  ARTIFICIAL

<220>
<223>  ASP-T5 AS

<400>  48
uugacacaug acaaaccuu                                                    19


<210>  49
<211>  19
<212>  DNA
<213>  ARTIFICIAL

<220>
<223>  ASP-T6 S

<400>  49
acuguguacu guutggaag                                                    19


<210>  50
<211>  19
<212>  DNA
<213>  ARTIFICIAL

<220>
<223>  ASP-T6 AS

<400>  50
ugacacauga caaaccuuc                                                    19


<210>  51
<211>  19
<212>  DNA
<213>  ARTIFICIAL

<220>
<223>  ASP-T7 S

<400>  51
cuguguacug uutggaagg                                                    19


<210>  52
<211>  19
<212>  DNA
<213>  ARTIFICIAL

<220>

<223>  ASP-T7 AS

<400>  52
gacacaugac aaaccuucc                                                    19


<210>  53
<211>  19
<212>  DNA
<213>  ARTIFICIAL

<220>
<223>  ASP-T8 S

<400>  53
uguguacugu utggaaggg                                                    19


<210>  54
<211>  19
<212>  DNA
<213>  ARTIFICIAL

<220>
<223>  ASP-T8 AS

<400>  54
acacaugaca aaccuuccc                                                    19


<210>  55
<211>  19
<212>  DNA
<213>  ARTIFICIAL

<220>
<223>  ASP-T9 S

<400>  55
guguacuguu tggaagggg                                                    19


<210>  56
<211>  19
<212>  DNA
<213>  ARTIFICIAL

<220>
<223>  ASP-T6

<400>  56
cacaugacaa accuucccc                                                    19


<210>  57
<211>  19
<212>  DNA
<213>  ARTIFICIAL

<220>
<223>  ASP-T10 S

<400>  57

uguacuguut ggaaggggu                                                    19

<210>  58
<211>  19
<212>  DNA
<213>  ARTIFICIAL

<220>
<223>  ASP-T10 AS

<400>  58
acaugacaaa ccuucccca                                                    19

<210>  59
<211>  19
<212>  DNA
<213>  ARTIFICIAL

<220>
<223>  ASP-T11 S

<400>  59
guacuguutg gaagggguu                                                    19

<210>  60
<211>  19
<212>  DNA
<213>  ARTIFICIAL

<220>
<223>  ASP-T11 AS

<400>  60
caugacaaac cuuccccaa                                                    19

<210>  61
<211>  19
<212>  DNA
<213>  ARTIFICIAL

<220>
<223>  ASP-T12 S

<400>  61
uacuguutgg aagggguuc                                                    19

<210>  62
<211>  19
<212>  DNA
<213>  ARTIFICIAL

<220>
<223>  ASP-T12 AS

<400>  62
augacaaacc uuccccaag                                                    19

<210>  63
<211>  19
<212>  DNA
<213>  ARTIFICIAL

<220>
<223>  ASP-T13 S

<400>  63
acuguutgga aggguucc                                                    19


<210>  64
<211>  19
<212>  DNA
<213>  ARTIFICIAL

<220>
<223>  ASP-T13 AS

<400>  64
ugacaaaccu uccccaagg                                                   19


<210>  65
<211>  19
<212>  DNA
<213>  ARTIFICIAL

<220>
<223>  ASP-T14 S

<400>  65
cuguutggaa gggguuccu                                                   19


<210>  66
<211>  19
<212>  DNA
<213>  ARTIFICIAL

<220>
<223>  ASP-T14 AS

<400>  66
gacaaaccuu ccccaagga                                                   19


<210>  67
<211>  19
<212>  DNA
<213>  ARTIFICIAL

<220>
<223>  ASP-T15 S

<400>  67
uguutggaag ggguuccuc                                                   19


<210>  68
<211>  19
<212>  DNA

<213>  ARTIFICIAL

<220>
<223>  ASP-T15 AS

<400>  68
acaaaccuuc cccaaggag                                                    19


<210>  69
<211>  19
<212>  DNA
<213>  ARTIFICIAL

<220>
<223>  ASP-T16 S

<400>  69
guutggaagg gguuccuca                                                    19


<210>  70
<211>  19
<212>  DNA
<213>  ARTIFICIAL

<220>
<223>  ASP-T16 AS

<400>  70
caaaccuucc ccaaggagu                                                    19


<210>  71
<211>  19
<212>  DNA
<213>  ARTIFICIAL

<220>
<223>  ASP-T17 S

<400>  71
uutggaaggg guuccucaa                                                    19


<210>  72
<211>  19
<212>  DNA
<213>  ARTIFICIAL

<220>
<223>  ASP-T17 AS

<400>  72
aaaccuuccc caaggaguu                                                    19


<210>  73
<211>  19
<212>  DNA
<213>  ARTIFICIAL

<220>

<223>  ASP-T18 S

<400>  73
utggaagggg uuccucaaa                                                    19


<210>  74
<211>  19
<212>  DNA
<213>  ARTIFICIAL

<220>
<223>  ASP-T18 AS

<400>  74
aaccuucccc aaggaguuu                                                    19


<210>  75
<211>  19
<212>  DNA
<213>  ARTIFICIAL

<220>
<223>  ASP-T19 S

<400>  75
tggaaggggu uccucaaau                                                    19


<210>  76
<211>  19
<212>  DNA
<213>  ARTIFICIAL

<220>
<223>  ASP-T19 AS

<400>  76
accuucccca aggaguuua                                                    19


<210>  77
<211>  19
<212>  DNA
<213>  ARTIFICIAL

<220>
<223>  SIRNA-REN (c+) S

<400>  77
ggccuuucac uacuccuac                                                    19


<210>  78
<211>  19
<212>  DNA
<213>  ARTIFICIAL

<220>
<223>  SIRNA-REN (C+) AS

<400>  78

ccggaaagug augaggaug 19

<210> 79
<211> 19
<212> DNA
<213> ARTIFICIAL

<220>
<223> SCAMBLE (c-) S

<400> 79
guauaauaca ccgcgcuac 19

<210> 80
<211> 19
<212> DNA
<213> ARTIFICIAL

<220>
<223> SCAMBLE (C-) AS

<400> 80
cauauuaugu ggcgcgaug 19

<210> 81
<211> 22
<212> DNA
<213> ARTIFICIAL

<220>
<223> PRIMER F

<400> 81
aaagggtcca tttgaggtta gg 22

<210> 82
<211> 23
<212> DNA
<213> ARTIFICIAL

<220>
<223> PRIMER R

<400> 82
tggtttattt accctcccct cct 23

<210> 83
<211> 21
<212> DNA
<213> ARTIFICIAL

<220>
<223> PRIMER

<400> 83
gaagaacttt tccaccagca g 21

<210>  84
<211>  22
<212>  DNA
<213>  ARTIFICIAL

<220>
<223>  PRIMER

<400>  84
tggtttattt accctccctc ct                                                22


<210>  85
<211>  33
<212>  DNA
<213>  ARTIFICIAL

<220>
<223>  PRIMER

<400>  85
gactgactct gaacttaata actgtgtact gtt                                    33


<210>  86
<211>  29
<212>  DNA
<213>  ARTIFICIAL

<220>
<223>  PRIMER

<400>  86
ctgactgact gacttgagga accccttcc                                         29

## Description

### FIELD OF THE INVENTION

The present invention concerns RNA interference mediated therapy for neurodegenerative diseases associated with lamin B1 protein overexpression.

### BACKGROUND OF THE INVENTION

Adult-onset autosomal dominant leukodystrophy (ADLD, OMIM#169500) (Eldridge et al. 1984) is a fatal disorder associated with CNS demyelination. ADLD is a slowly progressive disease caused by an excessive lamin B1 (LMNB1) production due to gene duplications (Giorgio et *al.* 2013; Padiath et *al.* 2006) or to an alteration in the lamin B1 regulatory landscape (Giorgio et al. 2015). ADLD onset is in the 4th/5th decade of life with autonomic symptoms, which precede cerebellar and pyramidal abnormalities by several years. Loss of fine motor control and gait disturbance appear later on and culminate in slowly progressive spastic quadriparesis, ataxia, and incontinence.

Pathogenic mechanisms in ADLD have only initially been explored. At the cellular level, LMNB1 regulates nuclear mechanics and integrity (Ferrera et al. 2014), interacts with chromatin determining chromosome segregation (Guelen et al. 2008), and regulates gene expression through mRNA synthesis (Tang *et al*. 2008) and splicing (Bartoletti-Stella *et al.* 2015; Camps *et al*. 2014).

All the pathogenic mechanisms proposed so far, are subsequent to *LMNB1* overexpression. It stands to reason that a genetic modulation of lamin B1 mRNA represents a promising strategy to treat ADLD.

In recent years, the use of synthetic siRNAs has become a popular choice for the development of genesilencing based therapies. A handful of siRNAs have now entered clinical trials, while others are in preclinical development.

Allele-Specific silencing by RNA Interference (ASP-iRNA) is a variant strategy that allows to downregulate a single mutant allele with minimal suppression of the corresponding wild type (WT) allele. ASP-iRNA was used to target autosomal dominant mutations or single nucleotide polymorphisms (SNPs) linked with aberrantly expanded trinucleotide repeats.

### OBJECT AND SUMMARY OF THE INVENTION

The object of the present invention is to identify siRNAs able to target lamin B1 allele that can be useful as therapeutic agents effective in the treatment of adult-onset autosomal dominant leukodystrophy (ADLD) associated with lamin B1 protein overexpression in a subject. The siRNAs object of the instant description are able to silence specifically one lamin B1 allele in the genome of the ADLD affected subject.

According to the invention, the above object is achieved thanks to the method specified in the ensuing claims, which are understood as forming an integral part of the present description.

In an embodiment, the instant disclosure discloses a siRNA targeting one lamin B1 allele, wherein the siRNA is selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

In a further embodiment, the present description discloses a therapeutic agent for use in the treatment of a subject affected by adult-onset autosomal dominant leukodystrophy (ADLD), wherein the therapeutic agent comprises one or more siRNAs targeting one lamin B1 allele, and wherein the one or more siRNAs are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

In a still further embodiment, the instant disclosure concerns a pharmaceutical composition comprising one or more siRNA, wherein the one or more siRNA are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand, and a pharmaceutically acceptable carrier.

The instant application discloses a method for the treatment of a patient affected by adult-onset autosomal dominant leukodystrophy comprising administering to the patient one or more siRNA, wherein the one or more siRNA are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

### BRIEF DESCRIPTION OF THE DRAWINGS

The invention will now be described, by way of example only, with reference to the enclosed figures of drawing, wherein:

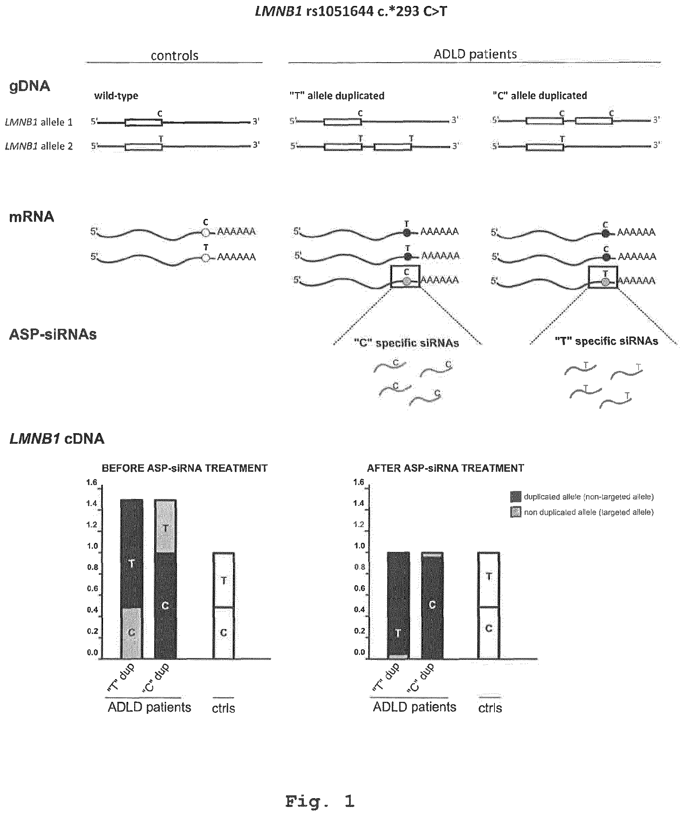

**FIGURE 1****. Rationale in the use of ASP-silencing in ADLD.** A frequent polymorphism located in the *LMNB1* gene (MAF=0.45) was identified. Six ADLD patients tested in the survey are heterozygous C/T: four patients carry the "T" allele duplicated and two the "C" allele duplicated of the SNP. At mRNA level, it was demonstrated that in ADLD patients the three LMNB1 alleles are equally expressed. Therefore, each allele contributes for one third to overall LMNB1 mRNA. The non-duplicated allele of the *LMNB1* gene was targeted by ASP-siRNAs with the aim of reducing expression close to a wild-type.

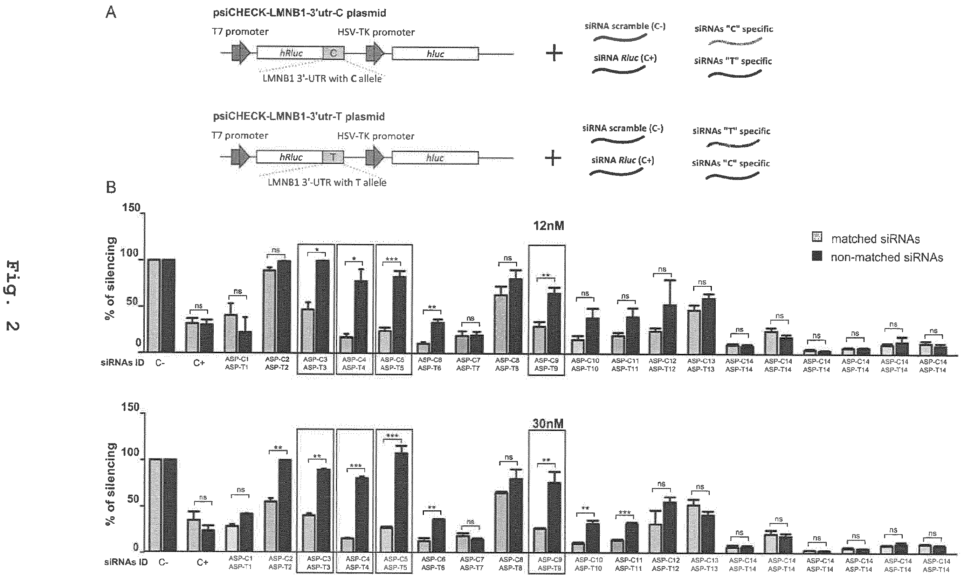

**FIGURE 2****. Identification of ASP-siRNAs using a custom dual-reporter system on HEK293T cells. A)** A dual reporter system was generated fusing the cLMNB1 to the *Renilla* (*hRluc*) gene after the translational stop. Firefly Luciferase *(hluc)* has been used as internal standard to normalize Renilla activity. Plasmid vectors have been co-transfected with each siRNAs [2 concentrations (12nM and 30nM)] into HEK293T cells. A siRNA targeting the Renilla and a nonspecific siRNA were used as controls. Luminescence was measured 48 hours post-transfection. **B**) At least 3 independent experiments have been performed. siRNAs "3,4,5 and 9" show high efficacy and allele selectivity and therefore have been considered positive hits (ASP-siRNAs) for further validations.

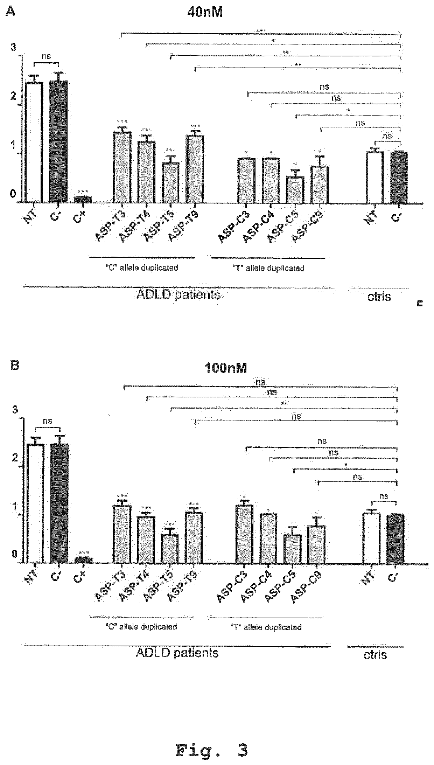

**FIGURE 3****. Validations of ASP-siRNAs on ADLD fibroblasts: evaluation of total mRNA levels.** Evaluation of total LMNB1 mRNA levels after 48h treatment with 40 (panel A) and 100nM (panel B) of siRNAs (*LMNB1* vs *HMBS,* Real Time PCR). Fold change and SEM are reported. In grey, statistical significance compared to scramble treatment of patients' cells (scramble (C-) column on the left). In black, statistical significance compared to scramble treatment of controls' cells (scramble (C-) column on the right). *<0.05; **< 0.02; ***<0.001 (Two-tailed, Mann-Whitney). Four different ADLD cell lines and three age-matched controls have been tested. At least three independent experiments have been performed. NT = not treated.

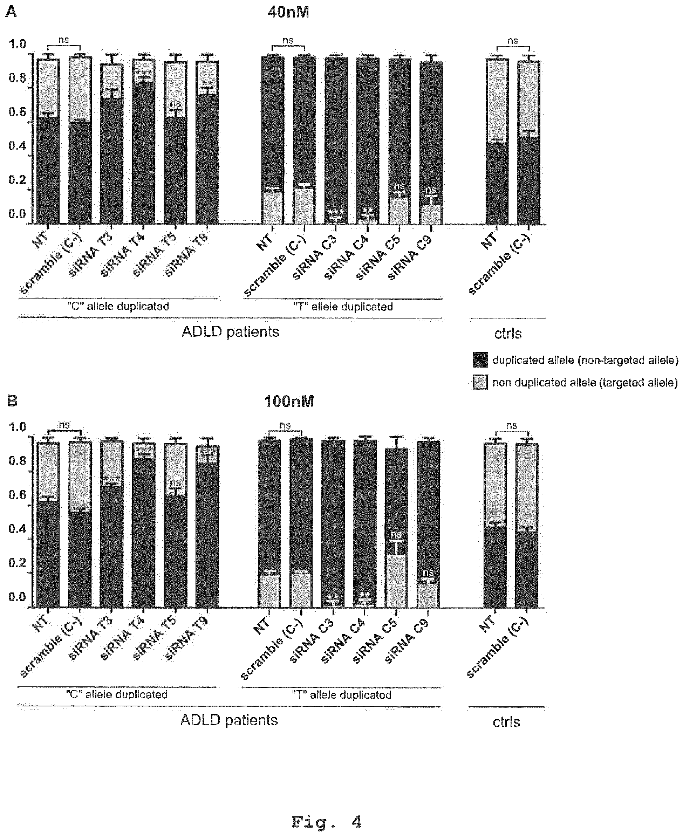

**FIGURE 4****. Validations of ASP-siRNAs on ADLD fibroblasts: evaluation of allele-specific mRNA levels.** Evaluation of allele specific expression after 48h treatment with 40 (panel A) and 100nM (panel B) of siRNAs (allele C versus allele T expression, Primer Extension Assay). Mean ± SEM of "C" versus "T" allele relative expressions are reported (C:T ratio). Statistical significance compared to scramble treatment of patients' cells was calculated using the Two-tailed, Mann-Whitney test. *<0.05; **< 0.02; ***<0.001. Four different ADLD cell lines and three age-matched controls have been tested. At least three independent experiment have been performed.

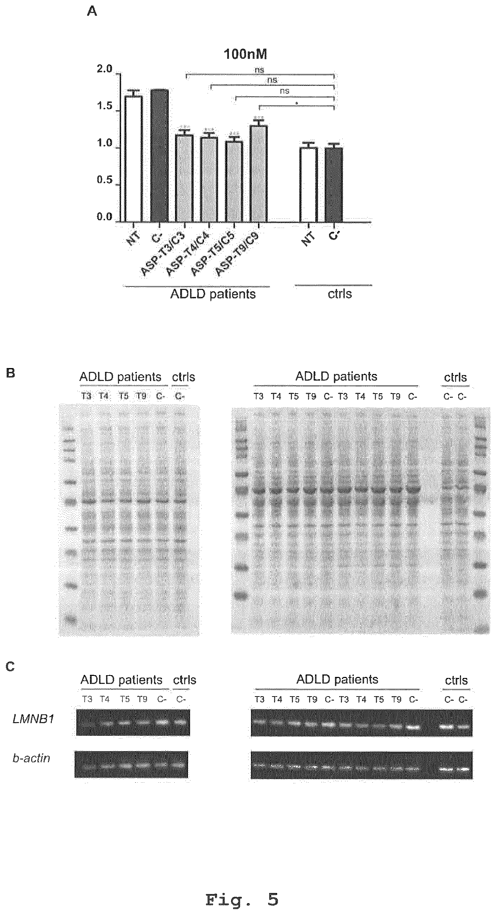

**FIGURE 5****. Validations of ASP-siRNAs on ADLD fibroblasts: evaluation of LMNB1 protein levels.** Evaluation of total LMNB1 protein after 48h treatment with 100nM of siRNAs (LMNB1 vs b-actin, western blot). Panel A: fold change and SEM are reported. In grey, statistical significance compared to scramble treatment of patients' cells (scramble (C-) column on the left). In black, statistical significance compared to scramble treatment of controls' cells (scramble (C-) column on the right). *<0.05; **< 0.02; ***<0.001 (Two-tailed, Mann-Whitney). Four different ADLD cell lines and three age-matched controls have been tested. At least three independent experiments have been performed. NT = not treated. Memcode staining (panel B) and immunostaining with anti-LMNB1 and anti-b actin antibodies (panel C) are shown as exemplification of the analysis.

### DETAILED DESCRIPTION OF THE INVENTION

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

The present description concerns a therapeutic agent for use in the treatment of adult-onset autosomal dominant leukodystrophy (ADLD) associated with lamin B1 protein overexpression, wherein the therapeutic agent comprises one or more siRNA targeting lamin B1 allele.

In an embodiment, the present description concerns siRNAs targeting one lamin B1 allele, wherein the siRNAs are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

In a further embodiment, the present description discloses a therapeutic agent for use in the treatment of a subject affected by adult-onset autosomal dominant leukodystrophy (ADLD), wherein the therapeutic agent comprises one or more siRNAs targeting one lamin B1 allele, and wherein the one or more siRNAs are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

In a preferred embodiment, the subject affected by ADLD carries three lamin B1 alleles and a single polynucleotide polymorphism (SNP) rs1051644 located in the lamin B1 gene having the sequence set forth in NM_005573.2 in heterozygous state, wherein among the three lamin B1 alleles two lamin B1 alleles are duplicated and one allele is non-duplicated with respect to the SNP rs1051644 of the lamin B1 gene.

In a still preferred embodiment, the one or more siRNA target the non-duplicated lamin B1 allele in the genome of the ADLD affected subject.

In a still further embodiment, the therapeutic agent for use in the treatment of a subject affected by adult-onset autosomal dominant leukodystrophy (ADLD) comprises one or more siRNAs selected from:
i) a double strand RNA composed of a sense strand comprising a base sequence set forth in SEQ ID No.: 5, 7, 9, and 17, and an antisense strand comprising a sequence complementary thereto, when the non-duplicated lamin B1 allele in the genome of the ADLD affected subject carries the "T" allele of the SNP rs1051644 of the lamin B1 gene having the sequence set forth in NM_005573.2; or
ii) a double strand RNA composed of a sense strand comprising a base sequence set forth in SEQ ID No.: 43, 45, 47, and 55, and an antisense strand comprising a sequence complementary thereto, when the non-duplicated lamin B1 allele in the genome of the ADLD affected subject carries the "C" allele of the SNP rs1051644 of the lamin B1 gene having the sequence set forth in NM_005573.2.

In an embodiment, the instant disclosure concerns a pharmaceutical composition comprising one or more siRNA, wherein the one or more siRNAs are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand, and a pharmaceutically acceptable carrier.

In a preferred embodiment the siRNA(s) is(are) incorporated into a vector suitable for administration to a human subject.

Allele-SPecific silencing by RNA Interference (ASP-iRNA) allows to specifically inhibit the expression of disease-causing alleles with minimal suppression of the corresponding wild-type alleles. This therapeutic strategy has been effectively used to target dominant activating mutations or SNPs *in cis* with aberrantly expanded trinucleotide repeats.

In the present application, the potential of ASP-iRNA as a molecular therapeutic approach for ADLD was investigated. Compared to other antisense strategies this approach allows the inhibition of one specific allele that is predicted to avoid excessive and potentially dangerous knockdown of the target gene. This latter point seems to be relevant for *LMNB1*, where excessive downregulation may have deleterious effects, as shown in cellular and mouse models (Bartoletti-Stella *et al.* 2015; Coffinier *et al.* 2010 and 2011; Giacomoni *et al*. 2016).

For ADLD, no treatment is available, and all medical interventions are palliative. Within the present description the first therapeutic option for ADLD based on Allele-SPecific (ASP) silencing by RNA interference (ASP-RNAi) is provided. As ADLD is caused by *LMNB1* gene duplication-mediated overexpression, the paramount choice for ADLD treatment would be a drug capable of restoring physiological levels of *LMNB1* expression without side effects of an excessive gene knockdown. This concern is critical for an exploitable therapy, because excessive *LMNB1* downregulation may have deleterious effects, as shown in cellular and mouse models.

ADLD patients have three, equally expressed, *LMNB1* alleles that can be discriminated exploiting the coding SNP rs1051644 in heterozygous state in the 50% of general population and in 80% of ADLD patients. Control subjects carry two LMNB1 alleles, one with the "C" and the other with the "T" allele of the SNP. ADLD patients have three LMNB1 copies: some patients carry two "C" alleles and one "T" allele, and others have two "T" alleles and one "C" allele, depending of which LMNB1 allele is duplicated.

The inventors of the present application chose to target the non-duplicated allele by ASP-siRNA, to silence specifically one of the three copies, with the aim of preserving the expression of two alleles, and in turn restoring physiological LMNB1 levels (Figure 1).

Because bioinformatics tools that can predict optimal conventional siRNAs from target gene sequences are less useful in predicting allele-specific targeting, a siRNA library centred on the SNP was designed and all the 19 possible siRNAs for each allele of the SNP were evaluated by using a customized dual reporter system. siRNAs have been co-transfected (two concentrations: 12.5 nM and 30nM) with the dual-reporter plasmids into HEK293T cells, and luminescence has been evaluated 48 hours post-transfection. Those siRNAs that selectively knockdown the target allele at the lower concentration, without affecting the other allele at the highest concentration have been considered as allele-specific siRNAs (ASP-siRNAs).

Four ASP-siRNAs with a high efficacy (p<0.0001) and allele-specificity (p<0.001) were identified being able to specifically silencing the target allele with minimal suppression of the non-target one (Figure 2).

These siRNAs were further tested in four ADLD patient-derived fibroblast lines. ADLD fibroblasts represent the ideal *in vitro* model for addressing the requirement for allele specificity. Indeed, they are not an overexpression model that fails to mimic the precise genomic context of the mutation found in patients and they show disease-associated phenotypes, whose rescue will further corroborate the therapeutic efficacy of ASP-silencing in ADLD. ADLD fibroblasts were transfected (40nM and 100 nM) with the four ASP-siRNAs, a scramble siRNA (C-) and a non-allele-specific siRNA against LMNB1 (C+) at two different concentrations, and LMNB1 mRNA and protein levels were evaluated 48 hours post-transfection. Real-time PCR allowed to evaluate total LMNB1 mRNA levels. As expected, ADLD fibroblast non-treated (NT) or treated with scramble siRNAs (C-) showed an increased expression of LMNB1 compare to control fibroblast (NT and C-) .

All the four ASP-siRNAs reduced LMNB1 expression and three (SNP position 3, 4, 9) restored physiological lamin B1 mRNA levels (Figure 3). To further demonstrate allele specificity of siRNAs, a primer extension assay was exploited as disclosed in Giorgio et al, 2013. This assay allowed to evaluate the relative contribution of each LMNB1 allele on total mRNA levels. NT ADLD fibroblasts or those treated with scramble siRNA showed a C:T ratio of about 65% (if the C allele of the SNP was duplicated) or 35% (if the T allele of the SNP was duplicated) versus a 50% of controls. The treatment with three ASP-siRNAs (3, 4, 9) specifically silenced the non-duplicated allele of the SNP moving the C:T ratio to 100% (in patients with the "C" allele duplicated) or 0% (in patients with the "T" allele duplicated) (Figure 4). These experiments demonstrated that three out of four ASP-siRNAs (3, 4 and 9) selectively silenced the target allele (p<0.0005) and restored *LMNB1* mRNA level to the wild type.

To further corroborate the therapeutic potential of ASP-siRNAs in ADLD, siRNAs ability to modulate LMNB1 protein level was evaluated. ADLD fibroblasts NT or treated with scramble siRNA showed an increased amount of Lamin B1 protein compare to controls. All the four ASP-siRNAs were able to reduce protein level and three siRNAs restore physiological LMNB1 amount (SNP position 3, 4, 5; Figure 5).

The results on patients' fibroblasts disclosed herein demonstrate that three siRNAs from the present library are able to knock down LMNB1 with an allele-specific action, reducing lamin B1 mRNA and protein to levels comparable with those of a healthy subject. These data strongly indicate that ASP-silencing is a therapeutic option for ADLD.

siRNA therapeutics are now well poised to enter clinics as a new class of drugs in the near future: at present more than 50 siRNAs are in clinical/preclinical development (Martinez et al., 2015), and a siRNA (Patisiran) for hereditary ATTR amylodosis is in a phase-III trial with promising results (Wittrup and Lieberman, 2015). In the last year, clinically relevant delivery of siRNAs has been achieved exploiting two main classes of delivery vehicles: non-viral (e.g., liposomes, exosomes, polymeric nanoparticles) or viral- (herpes simplex virus type 1, adenovirus, adeno-associated virus and lentivirus) vectors (Van den Boorn et al., 2011; Choudhury et al., 2016; Mishra et al., 2017). These systems allow to reach clinically efficient gene silencing of the non-duplicated *LMNB1* allele in the central nervous system (target tissue) of ADLD patients.

### MATERIALS AND METHODS

### Cell lines

Informed consent was collected from all participants of this project. The study has obtained ethical approval from the institutional review boards of the Alma Mater Studiorum, University of Bologna (Italy). Fibroblast cell lines were available for four ADLD patients (IT1, IT2, IT3, and BR1, see Giorgio et al., 2013) and three age matched controls. IT1, IT2, IT3 patients carry the duplication of the C-allele of the targeted SNP, whereas in BR1 patient the T-allele is duplicated.

The therapeutic strategy described here is applicable to subjects carrying: i) three *LMNB1* gene copies, and therefore presenting with Autosomal Dominant LeukoDystrophy (ADLD); and ii) the SNP rs1051644 (chr5:126172195, hg19) in heterozygous state.

Fibroblasts are primary cell lines that have limited lifespan. The immortalization of those cells is not recommended because it would alter the physiological characteristics of primary cells causing anomalies not related to the disease. For this reason, it is not possible storing ADLD fibroblasts in a cell bank. However, our ADLD cell lines will be available to the scientific community upon request.

A ploy to mimic the genomic contest of an ADLD fibroblastoid cell line might be generating an artificially LMNB1-duplicated cell line, by exploiting the CRISPR-Cas9 genome editing system. CRISPR-Cas9 is a recently developed technology that has revolutionized genome editing. It is a very efficient and easy method to create *in vitro* mutations and structural variations in the mammalian genome *(Ran et al., 2013).* This system is composed by a guide RNA (gRNA) and a DNA endonuclease (Cas9). The gRNA contains the hybridizing sequence, that specifically recognize a target region of the genome, and the Cas9-interacting regions, which allow the recruitment of the Cas9 endonuclease at the hybridization site where it creates a double-stranded break. A single guide RNA can be used to generate gene knockouts or to introduce specific mutations if combined with a donor sequence. Furthermore, the use of two guide RNAs allows more complex chromosomal rearrangements to be created such as deletions, inversions, and duplications (Li et al., 2015; Kraft et al., 2015).

Using a CRISPR/Cas9-based protocol (*Ran et al., 2013;* Kraft et al. 2015) scientists can generate the duplication of the *LMNB1* gene, starting from a control fibroblast cell line heterozygous for the SNP rs1051644. The resulting cell line will mimic the fibroblast cell lines from ADLD patients. In brief, two CRISPR guides (gRNA) will be designed to anneal a region upstream and downstream the *LMNB1* gene, respectively (hg19, chr5: 126112315-12617271; CRISPR design tool developed by (Hsu et al. 2013) and available at http://crispr.mit.edu/). To minimize off target effects, it is desirable to choose gRNAs with a quality score of above 85%. An all-in-one plasmid containing gRNAs, Cas9 and GFP coding sequences is required (Sigma-Aldrich). GFP-positive transfected cells have to be selected and single-cell sorted using a FACS system. Monoclonal cell lines have to be expanded and screened to identify correctly edited cells using a reverse primer (Rev) located at the 5' of the *LMNB1* gene and a forward primer (Fw) located at the 3' of the gene (see Li et al., 2015 for more details) . Correctly edited fibroblasts will be those cells showing PCR amplification products using the Fw and Rev primers.

### Constructs

The 3'-utr of the *LMNB1* gene (NM_005573.2) was PCR-amplified from a gDNA heterozygous for the targeted SNP using a forward primer containing a 5'-XhoI restriction site and a reverse primer containing a 5'-NotI restriction site under standard conditions (primers 5'-aaagggtccatttgaggttagg - SEQ ID No.: 81; and 5'-tggtttatttaccctcccctcct - SEQ ID No.:82). PCR amplification was performed in a total volume of 25 µl using 30 ng of genomic DNA, 200 µM dNTPs, 1X buffer A, 1 M betaine (B0300, Sigma-Aldrich), 1 unit of KAPA2G Fast (Kapa Biosystems). Thermal cycling conditions were: 1 min at 95°C, followed by 14 cycles of 10 sec at 95°C, 10 sec at annealing temperature 61°C-0.5°C/cycle and 1 sec at 72°C; then 25 cycles consisting of 10 sec at 95°C, 10 sec at 54°C, and 1 sec at 72°C. PCR products were gel purified with the GenEluate Gel Extraction kit (NA1111, Sigma-Aldrich), and inserted into the pGEM-T plasmid using the pGEM-T easy vector system I (A1360, Promega). Plasmids were transformed using the single-use JM109 competent cells (L2005, Promega), and extracted using the PureYield Plasmid Miniprep System (A1223, Promega). pGEM-T plasmids ("C" and "T" alleles) were digested with XhoI and NotI restriction endonucleases and each insert was gel-purified. The psiCHEK-LMNB1-3'UTR-C and psiCHEK-LMNB1-3'UTR-T expression constructs (from the psiCHECK™-2 system, C8021, Promega) were generated fusing the 3'UTR inserts ("C" and "T" alleles) to the *Renilla Luciferase* (*hRluc*) gene after the translational stop codon. *Firefly Luciferase (hluc+)* has been used as internal standard to normalize *Renilla* activity. Plasmids were transformed into single-use JM109 competent cells (L2005, Promega) and extracted using the PureYield Plasmid Midiprep System (A2495, Promega).

### siRNA design

A siRNA library with a 19+2 geometry targeting the "C" or the "T" alleles of the rs1051644 SNP was designed. The designed siRNA library with a 19+2 geometry (19-nucleotide duplex with a 3' dTdT nucleotide overhang) targeted the "C" and the "T" allele of the rs1051644. The sequence of the siRNAs are provided in Table 1 below. Because bioinformatics cannot optimally predict the best allele-specific siRNAs, all 19 possible siRNAs were considered (table 1). A siRNA targeting the *Renilla Luciferase* gene (*siRen,* C+) and a nonspecific siRNA (scramble, C-) were used as positive and negative controls, respectively. All siRNAs were synthesized by Eurofins Genomics (Ebersberg, Germany).

**Table 1: allele-specific siRNA library against rs1051644 (DNA variation c.*239C>T)**

| **siRNA name** | | **sequence 5'-3'** | **SEQ ID No:** | **siRNA name** | | **sequence 5'-3'** | **SEQ ID No:** |
|---|---|---|---|---|---|---|---|
| **ASP-C1** | **s** | UAAUAACUGUGUACUGUUC | 1 | **ASP-T1** | **s** | UAAUAACUGUGUACUGUUT | 39 |
| | **as** | AUUAUUGACACAUGACAAG | 2 | | **as** | AUUAUUGACACAUGACAAA | 40 |
| **ASP-C2** | **s** | AAUAACUGUGUACUGUUCG | 3 | **ASP-T2** | **s** | AAUAACUGUGUACUGUUTG | 41 |
| | **as** | UUAUUGACACAUGACAAGC | 4 | | **as** | UUAUUGACACAUGACAAAC | 42 |
| **ASP-C3** | **s** | ***AUAACUGUGUACUGUUCGG*** | ***5*** | **ASP-T3** | **s** | ***AUAACUGUGUACUGUUTGG*** | ***43*** |
| | **as** | ***UAUUGACACAUGACAAGCC*** | ***6*** | | **as** | ***UAUUGACACAUGACAAACC*** | ***44*** |
| **ASP-C4** | **s** | ***UAACUGUGUACUGUUCGGA*** | ***7*** | **ASP-T4** | **s** | ***UAACUGUGUA CUGUUTGGA*** | ***45*** |
| | **as** | ***AUUGACACAUGACAAGCCU*** | ***8*** | | **as** | ***AUUGACACAUGACAAACCU*** | ***46*** |
| **ASP-C5** | **s** | ***AACUGUGUACUGUUCGGAA*** | ***9*** | **ASP-T5** | **s** | ***AACUGUGUACUGUUTGGAA*** | ***47*** |
| | **as** | ***UUGACACAUGACAAGCCUU*** | ***10*** | | **as** | ***UUGACACAUGACAAACCUU*** | ***48*** |
| **ASP-C6** | **s** | ACUGUGUACUGUUCGGAAG | 11 | **ASP-T6** | **s** | ACUGUGUACUGUUTGGAAG | 49 |
| | **as** | UGACACAUGACAAGCCUUC | 12 | | **as** | UGACACAUGACAAACCUUC | 50 |
| **ASP-C7** | **s** | CUGUGUACUGUUCGGAAGG | 13 | **ASP-T7** | **s** | CUGUGUACUGUUTGGAAGG | 51 |
| | **as** | GACACAUGACAAGCCUUCC | 14 | | **as** | GACACAUGACAAACCUUCC | 52 |
| **ASP-C8** | **s** | UGUGUACUGUUCGGAAGGG | 15 | **ASP-T8** | **s** | UGUGUACUGUUTGGAAGGG | 53 |
| | **as** | ACACAUGACAAGCCUUCCC | 16 | | **as** | ACACAUGACAAACCUUCCC | 54 |
| **ASP-C9** | **s** | ***GUGUACUGUUCGGAAGGGG*** | ***17*** | **ASP-T9** | **s** | ***GUGUACUGUUTGGAAGGGG*** | ***55*** |
| | **as** | ***CACAUGACAAGCCUUCCCC*** | ***18*** | | **as** | ***CACAUGACAAACCUUCCCC*** | ***56*** |
| **ASP-C10** | **s** | UGUACUGUUCGGAAGGGGU | 19 | **ASP-T10** | **s** | UGUACUGUUTGGAAGGGGU | 57 |
| | **as** | ACAUGACAAGCCUUCCCCA | 20 | | **as** | ACAUGACAAACCUUCCCCA | 58 |
| **ASP-C11** | **s** | GUACUGUUCGGAAGGGGUU | 21 | **ASP-T11** | **s** | GUACUGUUTGGAAGGGGUU | 59 |
| | **as** | CAUGACAAGCCUUCCCCAA | 22 | | **as** | CAUGACAAACCUUCCCCAA | 60 |
| **ASP-C12** | **s** | UACUGUUCGGAAGGGGUUC | 23 | **ASP-T12** | **s** | UACUGUUTGGAAGGGGUUC | 61 |
| | **as** | AUGACAAGCCUUCCCCAAG | 24 | | **as** | AUGACAAACCUUCCCCAAG | 62 |
| **ASP-C13** | **s** | ACUGUUCGGAAGGGGUUCC | 25 | **ASP-T13** | **s** | ACUGUUTGGAAGGGGUUCC | 63 |
| | **as** | UGACAAGCCUUCCCCAAGG | 26 | | **as** | UGACAAACCUUCCCCAAGG | 64 |
| **ASP-C14** | **s** | CUGUUCGGAAGGGGUUCCU | 27 | **ASP-T14** | **s** | CUGUUTGGAAGGGGUUCCU | 65 |
| | **as** | GACAAGCCUUCCCCAAGGA | 28 | | **as** | GACAAACCUUCCCCAAGGA | 66 |
| **ASP-C15** | **s** | UGUUCGGAAGGGGUUCCUC | 29 | **ASP-T15** | **s** | UGUUTGGAAGGGGUUCCUC | 67 |
| | **as** | ACAAGCCUUCCCCAAGGAG | 30 | | **as** | ACAAACCUUCCCCAAGGAG | 68 |
| **ASP-C16** | **s** | GUUCGGAAGGGGUUCCUCA | 31 | **ASP-T16** | **s** | GUUTGGAAGGGGUUCCUCA | 69 |
| | **as** | CAAGCCUUCCCCAAGGAGU | 32 | | **as** | CAAACCUUCCCCAAGGAGU | 70 |
| **ASP-C17** | **s** | UUCGGAAGGGGUUCCUCAA | 33 | **ASP-T17** | **s** | UUTGGAAGGGGUUCCUCAA | 71 |
| | **as** | AAGCCUUCCCCAAGGAGUU | 34 | | **as** | AAACCUUCCCCAAGGAGUU | 72 |
| **ASP-C18** | **s** | UCGGAAGGGGUUCCUCAAA | 35 | **ASP-T18** | **s** | UTGGAAGGGGUUCCUCAAA | 73 |
| | **as** | AGCCUUCCCCAAGGAGUUU | 36 | | **as** | AACCUUCCCCAAGGAGUUU | 74 |
| **ASP-C19** | **s** | CGGAAGGGGUUCCUCAAAU | 37 | **ASP-T19** | **s** | TGGAAGGGGUUCCUCAAAU | 75 |
| | **as** | GCCUUCCCCAAGGAGUUUA | 38 | | **as** | ACCUUCCCCAAGGAGUUUA | 76 |
| | | | | | | | |
| **siRNA-*Ren* (C+)** | **s** | GGCCUUUCACUACUCCUAC | 77 | | | | |
| | **as** | CCGGAAAGUGAUGAGGAUG | 78 | | | | |
| **Scramble (C-)** | **s** | GUAUAAUACACCGCGCUAC | 79 | | | | |
| | **as** | CAUAUUAUGUGGCGCGAUG | 80 | | | | |

| | | | | | | | |
|---|---|---|---|---|---|---|---|
| Note: - All siRNA sequences carry a 2-bp overhang at their 3' (dTdT); - The underlined nucleotide corresponds to the SNP rs1051644 | | | | | | | |

### Cell Culture

The HEK293T cells and patients' derived fibroblasts were cultured in Dulbecco's modified Eagle's Medium (DMEM; 41965039, Life Technologies) with the addition of 10% fetal bovine serum (FBS; 10270-098, Life Technologies). All cultures were incubated at 37°C in the presence of 5% CO₂.

### Dual Luciferase Reporter Assay

HEK293T cells were seeded at a concentration of 2,5x10⁴ cells/well in a 96-well plate and incubated for 24 hours in DMEM supplemented with 5% FCS without antibiotics. Twenty nanograms of the psiCHEK-LMNB1-3'UTR-C or the psiCHEK-LMNB1-3'UTR-T vectors have been co-transfected with each siRNAs (2 concentrations: 12,5nM and 30nM) using Lipofectamine 2000 reagent (11668019, Life Technologies), following the manufacturer's protocol. Reporter was measured 48 hours post-transfection using the Dual-Luciferase® Reporter Assay System (E1910, Promega) on an analytical luminescence Glomax 20/20 luminometer (E5331, Promega). All 19 SNP-specific siRNAs, as well as si*Ren* and scramble siRNA were tested, evaluating their ability to silence specifically the target psiCHEK-LMNB1-3'UTR plasmid. For each assay, three independent experiments were performed.

### Validation of allele- specific siRNAs in ADLD fibroblasts.

ADLD and control fibroblasts were seeded at a concentration of 1.5x10⁵ cells/well in a 6-well plate and incubated for 24 hours in DMEM supplemented with 5% FCS without antibiotics. Cells were transfected with allele-specific siRNAs, a non-allele-specific LMNB1 siRNA (C+) and a scramble siRNA (AM 4620, Life Technologies; C-) at 40nM and 100nM concentrations. Forty-eight hours post transfection cells were harvested and split to extract RNA and proteins, respectively. For each siRNA, at least three independent experiments were performed.

### RNA isolation and quantitative Real Time PCR.

Total RNA was extracted from fibroblasts using the Direct-Zol RNA MiniPrep system (ZY-R2052, ZymoResearch) and cDNA was generated using the M-MLV Reverse Transcriptase kit (28025013, Life Technologies).

The expression levels of *LMNB1* and the reference gene hydroxymethylbilane synthase (*HMBS*) were measured with predesigned TaqMan assays (Applied Biosystems, *LMNB1*, Hs01059210_m1; *HMBS,* Hs00609297_m1).

Reactions were carried out in triplicate on an ABI 7500 real-time PCR machine (4366605, Applied Biosystem) using the ABI 2X TaqMan Universal PCR Master Mix II (4369016, Applied Biosystem), according to the manufacturer's instructions.

### Allelic Discrimination by Primer Extension Assay

To verify if one of the two *LMNB1* alleles was preferentially silenced, the relative amount of the two alleles of the targeted SNP was evaluated using a primer extension assay with the SNaPshot System (4323161, Applied Biosystems).

Primer extension was performed amplifying from cDNA with the primers and conditions reported in Giorgio et al., 2013. SNaPshot reactions were purified using Shrimp Alkaline Phosphatase (SAP, EF0651, Thermo Scientific), loaded on an ABI-Prism 3100 Avant capillary electrophoresis instrument with a GS120-Liz marker and analyzed using the GeneScan ver 3.7 software (Applied Biosystems).

### Western Blot analysis

Total proteins were extracted from fibroblasts in RIPA buffer. Seven µg of protein extracts were run on NuPAGE 4-12% Bis-tris Gel (NP0335BOX, Life Technologies), then blotted onto nitro-cellulose (162-0115, BIO-RAD) in Tris/Glycine buffer (28363, Thermo Scientific) with 20% methanol at 4°C for an hour and a half. Protein transfer efficiency was evaluated using the MemCode Reversible Protein Stain Kit (24580, Pierce Reversible Protein Stain Kit - for nitrocellulose membranes, Thermo Scientific). Lamin B1 and beta-actin were detected using primary anti-lamin B1 (ab16048, Abcam) and anti-b actin (ab8227, Abcam) antibodies and WesternBreeze™ Chemiluminescent Detection Kit (WB7106, Invitrogen). Images were captured with a ChemiDoc™ XRS+ System (Bio-Rad) and densitometric analysis was performed with Image Lab™ Software (Bio Rad).

### Statistical Analysis

Graphics and statistical analysis were performed with Graphpad Prism version 5.00 (Graphpad software). Data are presented as mean ± standard error of the mean (SEM) . All data were analyzed using Mann-Whitney t-*test.* Values are calculated relative to scramble siRNA (C-) using average of at least three independent experiments.

### RESULTS

### Identification of allele-specific and efficient siRNAs using a custom dual luciferase reporter system.

Bioinformatics tools that can predict optimal conventional siRNAs from target gene sequences are less useful in predicting allele-specific targeting (Hohjoh 2013). The inventors therefore designed and screened all the 19 possible siRNAs with a 19+2 geometry that could target the "C" or the "T" allele of the SNP rs1051644 (Table 1). A dual reporter assay (modified from the psiCHECK™-2 system, Promega; Figure 2) employing *Firefly* and *Renilla* luciferases was used to identify siRNAs with the highest ability for efficient and selective silencing of the target allele of the SNP. A siRNA targeting the *Renilla* luciferase gene and a nonspecific control siRNA have been used as positive and negative control, respectively (Table 1). In each case, *Renilla* activity was normalized against *Firefly* luciferase. The results of this screening are summarized in Figure 2 (panel B). Those siRNAs that selectively knockdown the target allele at the lower concentration, without affecting the other allele at the highest concentration have been considered as allele-specific siRNAs (ASP-siRNAs). Ten efficient but non-allele specific siRNAs were identified (SNP positions 1,7,12,13,14,15,16,17,18,19; Figure 2), as well as two non-efficient siRNAs (SNP positions 2 and 8; Figure 2) and five siRNAs showing allele specificity at least at the highest concentration tested (SNP position 3,4,5,6,9,10,11; Figure 2). Among these siRNAs the best four ASP-siRNAs (SNP positions 3,4,5,9; boxed in Figure 2) were selected for further validations.

### Validation of ASP-siRNAs on ADLD fibroblasts: mRNA level.

ADLD fibroblasts represent the ideal model to assess allele specificity of siRNAs because they present the precise genomic context of ADLD patients (Scholefield et al. 2014) and show disease-associated phenotypes (Ferrera et *al.* 2014; Bartoletti-Stella et al. 2015; Giorgio et al. 2015) required to corroborate ASP-silencing for ADLD therapy.

Four different ADLD cell lines (IT1, IT2, IT3 and BR1, Giorgio et al., 2013) have been used to validate ASP-siRNAs' ability to reduce *LMNB1* expression by real-time PCR (Figure 3). ADLD fibroblasts carrying the allele "C" of the SNP duplicated have been treated with ASP-siRNAs against the "T" allele of the SNP and *vice versa* (Figure 1). As expected, non-treated (NT) ADLD fibroblasts or those treated with a scramble siRNA (C-) showed an increase in *LMNB1* expression compared to the control samples [normalized *LMNB1* levels mean±SEM: (40nM: *patients* 2.472±0.176; *controls* 1.024±0.032; p<0.0001); (100nM: *patients* 2.452±0.171; *controls* 1.015±0.031; p<0.0001)]. From the library a highly efficient but non-allele specific siRNA (SNP position 16, figure 2) was chosen to evaluate efficacy of silencing in the experiments (C+ in figure 3). Each ASP-siRNA tested reduces *LMNB1* mRNA levels compared to scramble siRNAs both at 40nM and 100nM of final concentrations (*"C"-duplicated patients:* p<0.0005; "*T*"-*duplicated patient* p<0.05). Interestingly, three out of four ASP-siRNAs (SNP position 3, 4 and 9) restore *LMNB1* expression to physiological levels at the highest concentration tested, suggesting the efficacy and allele specificity of these three siRNAs [normalized *LMNB1* levels mean±SEM: *("C"-duplicated patients:* T3 1.189±0.117; T4 0.963±0.081; T9 1.052±0.094; *controls* 1.015±0.031; p=ns); *"T"-duplicated patients:* C3 1.207±0.105; C4 1.035±0.007; C9 0.785±0.185; *controls* 1.015±0.031; p=ns)]. At the same concentration the C+ siRNAs reduced LMNB1 expression close to zero (normalized *LMNB1* levels mean±SEM: 0.085±0.015), further corroborating the allele-specificity of the ASP-siRNAs identified.

To evaluate allele-specificity of the four ASP-siRNAs identified, we evaluated the relative amount of the two LMNB1 alleles of the targeted SNP using a primer extension assay with the SNaPshot System (Applied Biosystems, Foster City, CA, USA). Primer extension was performed from cDNA with the primers and conditions reported in Giorgio et al., 2013. In brief, a 394 bp fragment ws amplified using primers 5'-gaagaacttttccaccagcag (SEQ ID No.: 83) and 5'-tggtttatttaccctccctcct (SEQ ID No.: 84) and the KAPA2G Fast system (Kapa Biosystems. PCR products were purified using Exonuclease I and Shrimp Alkaline Phosphatase and the primer extension reaction performed with primers annealing immediately before and after the SNP base (5'-gactgactctgaacttaataactgtgtactgtt - SEQ ID No.: 85, 5'- ctgactgactgacttgaggaaccccttcc - SEQ ID No.: 86). SNaPshot reactions were purified using Shrimp Alkaline Phosphatase (SAP) and loaded on an ABI-Prism 3100 Avant capillary electrophoresis instrument (Applied Biosystems). The relative contribution of the non-duplicated (matched) and the duplicated (non-matched) LMNB1 alleles before and after ASP-siRNAs' treatment was determined (Figure 4). As expected the matched allele almost completely silenced after siRNAs treatment, with a C:T ratio of LMNB1 alleles close to 1 in the ADLD patients with the "C" allele duplicated and close to 0 in patients with the "T" allele duplicated. ASP-siRNAs T3, T4 and T9 preferentially silenced the matched allele at both concentrations tested, with the best allele specificity at 100nM (LMNB1 alleles C:T ratio mean±SEM: C- 0.601±0.024; T3 0.712±0.019; T4 0.872±0.029; T9 0.847±0.050; p<0.0005). Whereas only C3 and C4 siRNAs showed a strong allele specificity for the matched LMNB1 allele (LMNB1 C:T alleles ratio at 100nM mean±SEM: C- 0.217±0.024; C3 0.018±0.018; C4 0.024±0.024; p<0.0001). As expected based on real-time results both siRNAs with the SNP at position 5 (T5 and C5 in figure 4) did not shown a statistically significant allele-specificity [LMNB1 C:T alleles ratio at 100nM mean±SEM: (C- 0.601±0.024; T5 0.655±0.047; p=ns) (C- 0.217±0.017; C5 0.317±0.084; p=ns). The primer extension results corroborate the allele specificity and efficiency of siRNAs T3, T4, T9 for the treatment of ADLD fibroblasts with the "C" allele of the SNP duplicated and of siRNAs C3 and C4 for those carrying the duplication of the allele "T".

### Validation of ASP-siRNAs on ADLD fibroblasts: protein level.

To further corroborate ASP-siRNA efficacy as therapeutic strategy for ADLD, the inventors verified if the LMNB1 silencing mediated by ASP-siRNAs at LMNB1 mRNA level is correlated to a reduction at protein level. All treatments tested (ASP-siRNAs SNP positions 3,4,5 and 9, concentration of 100nM final) are associated to a statistically significant reduction of LMNB1 protein compare scramble siRNA (ADLD patients, normalized LMNB1 protein mean±SEM: C- 1.777±0.06; T3 1.167±0.072 T4 1.149±0.063; T5 1.085±0.066; T9 1.299±0.077; p<0.0001). Most interestingly, the reduction mediated by ASP-siRNAs 3, 4 and 5 restore LMNB1 protein to control values (Figure 5).

### REFERENCES

Bartoletti-Stella A, Gasparini L, Giacomini C, Corrado P, Terlizzi R, Giorgio E, Magini P, Seri M, Baruzzi A, Parchi P, Brusco A, Cortelli P, Capellari S. 2015. Messenger RNA processing is altered in autosomal dominant leukodystrophy. Hum Mol Genet 24:2746-2756.
Camps J, Wangsa D, Falke M, Brown M, Case CM, Erdos MR, Ried T. 2014. Loss of lamin B1 results in prolongation of S phase and decondensation of chromosome territories. FASEB J 28:3423-3434.
Choudhury SR, Hudry E, Maguire CA, Sena-Esteves M, Breakefield XO, Grandi P. 2016. Viral vectors for therapy of neurologic diseases. Neuropharm. 120:63-80.
Coffinier C, Chang SY, Nobumori C, Tu Y, Farber EA, Toth JI, Fong LG, Young SG. 2010. Abnormal development of the cerebral cortex and cerebellum in the setting of lamin B2 deficiency. Proc Natl Acad Sci U S A 107:5076-5081.
Coffinier C, Jung HJ, Nobumori C, Chang S, Tu Y, Barnes RH, 2nd, Yoshinaga Y, de Jong PJ, Vergnes L, Reue K, Fong LG, Young SG. 2011. Deficiencies in lamin B1 and lamin B2 cause neurodevelopmental defects and distinct nuclear shape abnormalities in neurons. Mol Biol Cell 22:4683-4693.
Eldridge R, Anayiotos CP, Schlesinger S, Cowen D, Bever C, Patronas N, McFarland H. 1984. Hereditary adult-onset leukodystrophy simulating chronic progressive multiple sclerosis. N Engl J Med. 311(15):948-53.
Ferrera D, Canale C, Marotta R, Mazzaro N, Gritti M, Mazzanti M, Capellari S, Cortelli P, Gasparini L. 2014. Lamin B1 overexpression increases nuclear rigidity in autosomal dominant leukodystrophy fibroblasts. FASEB J 28:3906-3918.
Giacomini C, Mahajani S, Ruffilli R, Marotta R, Gasparini L. 2016. Lamin B1 protein is required for dendrite development in primary mouse cortical neurons. Mol Biol Cell 27:35-47.
Giorgio E, Robyr D, Spielmann M, Ferrero E, Di Gregorio E, Imperiale D, Vaula G, Stamoulis G, Santoni F, Atzori C, Gasparini L, Ferrera D, Canale C, Guipponi M, Pennacchio LA, Antonarakis SE, Brussino A, Brusco A. 2015. A large genomic deletion leads to enhancer adoption by the lamin B1 gene: a second path to autosomal dominant adult-onset demyelinating leukodystrophy (ADLD). Hum Mol Genet 24:3143-3154.
Giorgio E, Rolyan H, Kropp L, Chakka AB, Yatsenko S, Di Gregorio E, Lacerenza D, Vaula G, Talarico F, Mandich P, Toro C, Pierre EE, Labauge P, Capellari S, Cortelli P, Vairo FP, Miguel D, Stubbolo D, Marques LC, Gahl W, Boespflug-Tanguy O, Melberg A, Hassin-Baer S, Cohen OS, Pjontek R, Grau A, Klopstock T, Fogel B, Meijer I, Rouleau G, Bouchard JP, Ganapathiraju M, Vanderver A, Dahl N, Hobson G, Brusco A, Brussino A, Padiath QS. 2013. Analysis of LMNB1 duplications in autosomal dominant leukodystrophy provides insights into duplication mechanisms and allele-specific expression. Hum Mutat 34:1160-1171.
Guelen L, Pagie L, Brasset E, Meuleman W, Faza MB, Talhout W, Eussen BH, de Klein A, Wessels L, de Laat W, van Steensel B. 2008. Domain organization of human chromosomes revealed by mapping of nuclear lamina interactions. Nature 453:948-951.
Hsu PD, Scott DA, Weinstein JA, Ran FA, Konermann S, Agarwala V, Li Y, Fine EJ, Wu X, Shalem O, Cradick TJ, Marraffini LA, Bao G, Zhang F. 2013. DNA targeting specificity of RNA-guided Cas9 nucleases. Nat. Biotechnol. 31: 827-832.
Kraft K, Geuer S, Will AJ, Chan WL, Paliou C, Borschiwer M, Harabula I, Wittler L, Franke M, Ibrahim DM, Kragesteen BK, Spielmann M, Mundlos S, Lupiáñez DG, Andrey G. 2015. Deletions, Inversions, Duplications: Engineering of Structural Variants using CRISPR/Cas in Mice. Cell Rep. 1247(15)00029-7.
Li Y, Park AI, Mou H, Colpan C, Bizhanova A, Akama-Garren E, Joshi N, Hendrickson EA, Feldser D, Yin H, Anderson DG, Jacks T, Weng Z, Xue W. 2015. A versatile reporter system for CRISPR-mediated chromosomal rearrangements. Genome Biol. 28; 16:111
Mishra DK, Balekar N, Mishra PK. 2107. Nanoengineered strategies for siRNA delivery: from target assessment to cancer therapeutic efficacy. Drug Deliv and Transl. Res. 7:346-358.
Padiath QS, Saigoh K, Schiffmann R, Asahara H, Yamada T, Koeppen A, Hogan K, Ptacek LJ, Fu YH. 2006. Lamin B1 duplications cause autosomal dominant leukodystrophy. Nat Genet 38:1114-1123.
Ran FA, Hsu PD, Wright J, Agarwala V, Scott DA, Zhang F. 2013. Genome engineering using the CRISPR-Cas9 system. Nat Protoc. (11): 2281-2308.
Scholefield J, Watson L, Smith D, Greenberg J, Wood MJ. 2014. Allele-specific silencing of mutant Ataxin-7 in SCA7 patient-derived fibroblasts. Eur J Hum Genet 22:1369-1375.
Tang CW, Maya-Mendoza A, Martin C, Zeng K, Chen S, Feret D, Wilson SA, Jackson DA. 2008. The integrity of a lamin-B1-dependent nucleoskeleton is a fundamental determinant of RNA synthesis in human cells. J Cell Sci 121:1014-1024.
Van den Boorn JG, Schlee M, Coch C, Hartmann G. 2011. SiRNA delivery with exosome nanoparticles. Nat Biotech 29: 325-326.
Wittrup A, Lieberman J. 2015. Knocking down disease: a progress report on siRNA therapeutics. Nat Rev Genet 16:543-552.

## Claims

1. siRNA targeting lamin B1 allele, wherein the siRNA is selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

2. Therapeutic agent for use in the treatment of a subject affected by adult-onset autosomal dominant leukodystrophy (ADLD), wherein said therapeutic agent comprises one or more siRNAs targeting one lamin B1 allele, and wherein the one or more siRNAs are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

3. Therapeutic agent for use according to claim 2, wherein the one or more siRNAs have the overhang at the 3' terminal of the sense strand.

4. Therapeutic agent for use according to claims 2 or 3, wherein the subject affected by ADLD carries three lamin B1 alleles and a single nucleotide polymorphism (SNP) rs1051644 located in the lamin B1 gene having the sequence set forth in NM_005573.2 in heterozygous state, wherein among the three lamin B1 alleles two lamin B1 alleles are duplicated and one allele is non-duplicated with respect to the SNP rs1051644.

5. Therapeutic agent for use according to any one of claims 2 to 4, wherein the one lamin B1 allele targeted by the one or more siRNAs is the non-duplicated lamin B1 allele.

6. Therapeutic agent for use according to claims 4 or 5, wherein the one or more siRNAs are selected from:
i) a double strand RNA composed of a sense strand comprising a base sequence set forth in SEQ ID No.: 5, 7, 9, and 17, and an antisense strand comprising a sequence complementary thereto, when the non-duplicated lamin B1 allele carries the "T" allele of the SNP rs1051644 of the lamin B1 gene having the sequence set forth in NM_005573.2; or
ii) a double strand RNA composed of a sense strand comprising a base sequence set forth in SEQ ID No.: 43, 45, 47, and 55, and an antisense strand comprising a sequence complementary thereto, when the non-duplicated lamin B1 allele carries the "C" allele of the SNP rs1051644 of the lamin B1 gene having the sequence set forth in NM_005573.2.

7. Therapeutic agent for use according to any one of claims 2 to 6, wherein the siRNA is comprised in a delivery system, wherein the delivery system is suitable for administration to a human being, preferably the delivery system is selected from viral and non-viral vectors.

8. Pharmaceutical composition comprising one or more siRNAs and a pharmaceutically acceptable carrier, wherein the one or more siRNAs are selected from a double strand RNA composed of a sense strand comprising a base sequence set forth in SEQ ID No.: 5, 7, 9, 17, 43, 45, 47, and 55 and an antisense strand comprising a sequence complementary thereto, which optionally has an overhang at the terminal of the sense and/or antisense strand.

9. Pharmaceutical composition according to claim 8, wherein each siRNA is comprised in a vector, wherein the vector is suitable for administration to a human being.
